(19) Europäisches Patentamt / European Patent Office / Office européen des brevets

(11) **EP 2 737 908 B1**

(12) **EUROPEAN PATENT SPECIFICATION**

(45) Date of publication and mention
of the grant of the patent:
**11.03.2015 Bulletin 2015/11**

(51) Int Cl.:
*A61K 45/06* (2006.01)     *A61K 31/728* (2006.01)
*A61K 31/738* (2006.01)     *A61P 27/06* (2006.01)

(21) Application number: **13194011.6**

(22) Date of filing: **22.11.2013**

(54) **Viscoelastic gels in ophthalmic surgery**

Viskoelastische Gele in der Augenchirurgue

Gels viscoélastiques en chirurgie opthalmique

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.11.2012 IT PD20120360**

(43) Date of publication of application:
**04.06.2014 Bulletin 2014/23**

(73) Proprietor: **FIDIA FARMACEUTICI S.p.A.
35031 Abano Terme (PD) (IT)**

(72) Inventors:
• **Renier, Davide
35031 Abano Terme (PD) (IT)**
• **Guarise, Cristian
35031 ABANO TERME (PD) (IT)**

(74) Representative: **Minoja, Fabrizio
Bianchetti Bracco Minoja S.r.l.
Via Plinio, 63
20129 Milano (IT)**

(56) References cited:
**EP-A1- 0 341 745       WO-A1-2007/006484
WO-A2-2011/023355    US-A1- 2010 069 938**

• Foscarini B. et al: **"A comparative study between crosslinked sodium hyaluronate (Healaflow) and sodium hyaluronate 2.3 (Healon 5) as subconjunctival spacers injected into failed filtration blebs during needling with MMC", , 2 August 2012 (2012-08-02), XP002702827, Retrieved from the Internet: URL:http://www.oic.it/~egscopenaghen2012/p osters/june20/P5.12/poster.pdf [retrieved on 2013-07-16]**

## Description

## FIELD OF INVENTION

The present invention relates to viscoelastic gels for applications in ophthalmic surgery.

## TECHNOLOGICAL BACKGROUND

Aqueous humour is a transparent liquid produced by the ciliary body; it wets the internal structures of the eye, lubricating and partly nourishing them, and, together with the vitreous humour, keeps the eyeball "swollen". It is then drained by various mechanisms, the most important of which is trabecular outflow; the trabecular meshwork is a spongy structure located along the entire circumference of the eye, in the space between the outermost part of the cornea and the outermost part of the iris.

The other drainage routes are uveoscleral flow and posterior flow (resorption through a pump system by the vitreous humour, retina and retinal pigment epithelium). Once produced, the aqueous humour flows into the posterior chamber (the space between the iris and the lens) and from there, through the pupil, passes into the anterior chamber (the space between the iris and the cornea), to reach the angle chamber and the trabecular meshwork.

The vitreous humour and aqueous humour contribute to generating the inner tension of the eye and, as the vitreous humour does not change volume in the individual's lifetime, oscillations in ocular pressure mainly depend on the balance between production and drainage of the aqueous humour. The ocular pressure therefore rises as a result of both increased production and reduced outflow of aqueous humour, but it is the latter case which has the greatest clinical importance.

Glaucoma can be defined as "a degenerative disease of the optic nerve with a multifactorial origin", because various forms of glaucoma exist. The most common involves an increase in intraocular pressure; in other forms, the trigger factor is weakness of the optic nerve due to vascular or structural causes.

The optic nerve consists of 1,200,000 fibres that exit from the eyeball through a circular opening in the fundus. At this point, the fibres bend by 90°, and the increased ocular tension can easily push and squeeze them towards the edge of the scleral foramen. Strangulation of the fibres causes them to fail, and for each group of interrupted fibres, a corresponding area of lower visual sensitivity appears in the field of vision. The optic nerve is eroded from the inside, and its central excavation is enlarged. When high ocular pressure leads to erosion of the optic nerve and the appearance of an impaired field of vision, the disorder is called glaucomatous disease or glaucoma.

The classification of the various types of glaucoma is complex, but a distinction can basically be made between angle-closure glaucoma and open-angle glaucoma.

In angle-closure glaucoma, the ocular pressure suddenly rises from normal values (below 18 mmHg) to very high values (40-50 mmHg and over), whereas in open-angle glaucoma the increase in pressure is slow and gradual; in the first case, the symptoms are evident (eye pain, red eye and reduced visual acuity, eyeball with a very hard, stony texture), whereas in the second, the patient is essentially asymptomatic.

When treating an acute attack of glaucoma, it is important to reduce the pressure rapidly with a local treatment (e.g. with eyedrops containing beta-blockers, alpha-adrenergic or cholinergic agonists based on pilocarpine) and systemic treatment (mannitol infusion) to relieve the compression of the optic nerve; it is also essential to decongest the eyeball with cortisone and anti-inflammatories.

Argon laser iridectomy and YAG laser iridotomy are parasurgical out-patient treatments which breach the iris, thus creating an alternative route for the circulation of aqueous humour in addition to the normal passage through the pupillary foramen. Argon Laser Trabeculoplasty (ALT) and its variant Selective Laser Trabeculoplasty (SLT) create holes in the trabecular meshwork to facilitate the passage of the aqueous humour through said meshwork.

There are two types of surgical treatment of glaucoma: perforating and non-perforating surgery, and operations to position drainage implants.

Perforating surgery (trabeculectomy) involves creating a breach/fistula in the wall of the eye which allows the direct passage of aqueous humour from the anterior chamber to the subconjunctival space. A basal iridectomy is also performed at the surgical fistula. This type of surgery very often involves adverse events, which can be serious, and its success depends on the patency of the fistula and the creation of a conjunctival filtering bleb. However, the excessive, incorrect healing process of the tissues concerned often prejudices the result of the operation, and the sclera area in which the operation is performed (specifically the surgical aperture and the subconjunctival space) must necessarily be pre-treated with antimetabolites such as 5-fluorouracil or mitomycin-C (MMC) to reduce the proliferation of fibroblasts and vascular cells, which would otherwise obstruct the surgical breach, thus requiring further surgery (such as needling of the bleb).

5-Fluorouracil and mitomycin-C are potentially toxic antitumoral drugs; many post-operative complications are attributable to them, such as ocular hypotony, epithelial toxicity (of fluorouracil), toxicity to the ciliary body (of MMC) and finally, an increased risk of postoperative endophthalmitis.

Non-perforating surgery is used to increase the controlled filtration mechanism. Viscocanalostomy is designed to restore the outflow through the natural routes (Schlemm's channel, collector channels and episcleral veins), whereas in deep sclerectomy, filtration is also created in the subconjunctival space. In glaucoma surgery, substances designed to keep the filtration spaces pervious are normally used; in trabeculectomy, viscocanalostomy and sclerectomy, polymers such as high-molecular-weight hyaluronic acid (HA), possibly combined with collagen, are injected, positioned under the scleral flap to keep the decompression chamber/filtration bleb pervious, and promote subconjunctival filtration.

Hyaluronic acid (HA) is a heteropolysaccharide consisting of alternating residues of D-glucuronic acid and N-acetyl-D-glucosamine. It is a straight-chain polymer with a molecular weight ranging between 50,000 and $13 \times 10^6$ Da, depending on the source from which it is obtained and the preparation methods used. It is present in nature in pericellular gels, in the ground substance of the connective tissue of vertebrates, in the vitreous humour and in the umbilical cord.

HA plays an important part in the biological organism as a structural and mechanical support for the tissues, and as an active component in the cell physiology of tissues such as skin, tendons, muscles and cartilage.

HA also plays a crucial role in the tissue repair process both from the structural standpoint and as a substance that stimulates/regulates a wide range of physiological processes in which said polysaccharide is directly and/or indirectly involved. It is known that hyaluronic acid is used not only in tissue healing, but also as a filler in cosmetic skin surgery and as a viscosupplement in the treatment of osteoarthritis, because it is immunologically inert, non-toxic, biodegradable and bioresorbable.

The products currently on the market for use in the glaucoma surgery described above are based on unmodified HA (Healon 5, Pharmacia) and HA crosslinked with BDDE (1,4-butanediol diglycidyl ether) (HEALA-flow®, ANTEIS, US20100069938). The two products have demonstrated equal efficacy when used and compared in glaucoma surgery combined with mitomycin C (MMC) (Foscarini B. et al., ARVO 2012 Annual Meeting Abstracts).

The present invention relates to viscoelastic gels for applications in ophthalmic surgery comprising HA crosslinked in different ways, so as to confer *in vivo* an antifibrotic capacity and breakdown time greater than those of the reference products specified above.

**DETAILED DESCRIPTION OF THE INVENTION**

The present invention relates to viscoelastic gels formed by mixing two HA derivatives crosslinked in different but complementary ways (ACP and HBC), previously known as fillers for use in dermocosmetology (EP2470230), for use in ophthalmic surgery, in particular in all types of glaucoma surgery, whether perforating or non-perforating, in the technique called "needling of the bleb" (which is necessary in the case of post-surgical fibrosis), or for the positioning of drainage implants.

The viscoelastic gels of the invention ensure:

- the patency of the drainage channels and aqueous humour filtration bleb filled surgically by said gels, as their degradation times *in vivo* have proved very long, and longer than those of comparative HA gels crosslinked with BDDE;
- the correct filtration function of said channels and filtration bleb, as the gels resulting from mixing said HA derivatives are single-phase viscoelastic gels whose rheological characteristics the Applicant demonstrates below, and which are wholly comparable to those of the products based on HA crosslinked with 1,4-butanediol diglycidyl ether (BDDE) used in glaucoma surgery;
- a reduction in fibrosis of the scleral area subjected to ophthalmic surgery, in particular collateral to glaucoma surgery, as they significantly reduce (compared with comparative HA gels crosslinked with BDDE) the proliferation of the cells in the scleral area concerned, but without being cytotoxic, thus enabling the surgeon to use said gels without simultaneously treating the surgical zone with antiproliferative drugs, or in any event allowing them to be reduced in the ways and by the quantities required to achieve the desired result.

Said viscoelastic gels (as described in EP2470230) are formed by:

- autocrosslinked hyaluronic acid (ACP) mixed with
- hyaluronic acid crosslinked with BDDE (HBC).

The ACP used in the present invention, prepared as described in EP0341745, has an average degree of crosslinking ranging from 4 to 5%, and is preferably prepared with HA having a weight-average molecular weight (MW) of 200 KDa. When hydrated, it presents as an autocrosslinked gel, with no molecules foreign to the native polysaccharide, as it is based on the ester bond between the carboxyl and hydroxyl groups of the same polysaccharide chain and/or the adjacent chains. It is therefore devoid of immunotoxicity, as biocompatible as native HA, highly hydrating, and easily degradable by hyaluronidase.

Conversely, HA crosslinked with BDDE (a molecule containing epoxy groups for the formation of ethers on the primary hydroxyls of HA) contains the crosslinking molecule, and is therefore more resistant to enzymatic degradation as it possesses ether bonds that stabilise the polysaccharide, giving the product obtained a long residence time *in situ*.

The HBC according to the invention has a BDDE/HA molar ratio falling into the range from 2 to 7 moles of BDDE per 100 moles (2% - 7% moles/mole) of HA dimer (i.e. D-glucuronic acid and N-acetyl-D-glucosamine), preferably from 4% to 5% moles/mole of dimer, and is prepared as described in EP2470230 (preferably according to preparation process B) and Examples 1-3.

The HA used in the present invention to prepare the derivatives described above preferably takes the form of sodium salt, can be obtained from any source, such as extraction from rooster combs (EP0138572) or fermentation (e.g. from *Streptococcus equi,* as known to the skilled person), or by technological means (e.g. from *Bacillus,* WO2012/032154), and has a weight-average molecular weight (MW) (determined by the limiting viscosity number method: Terbojevich et al., Carbohydrate Research, 1986, 149:363-377) ranging from 400 to $3\times10^6$Da, preferably from $1\times10^5$Da to $1\times10^6$Da, most preferably from 200,000 to 750,000 Da.

The total quantity of HA present in the gels of the invention, in the form of both ACP and HBC, can range from 10 to 40 mg/ml, preferably from 20 to 30 mg/ml, prepared in the form of a viscoelastic gel with pharmaceutically acceptable carriers and/or excipients (such as saline and physiological solutions consisting of NaCl and/or phosphate salts; said gels are consequently hydrogels), as they maintain the desired osmolarity and pH value (generally between 5.5 and 7).

The chemically heterogeneous nature of the viscoelastic gel (ACP+HBC) allows the properties of the end product to be modulated by suitably varying the weight ratio between the components. The two HAs can be mixed in an ACP:HBC weight ratio ranging from 5:95 to 50:50, preferably from 5:95 to 25:75, most preferably can be 25:75 of ACP:HBC; the weight ratio will be selected according to the desired final viscosity, which will depend on the type of ophthalmic surgery performed.

The mixing of the two crosslinked substances leads to the formation of a single-phase viscoelastic gel, with characteristics of biocompatibility comparable with those of native hyaluronic acid, but with a different biodegradability so that, when implanted *in vivo,* its residence time *in situ* is much longer than that of unmodified HA or the gels formed by HA crosslinked with BDDE currently used in ophthalmic surgery, as shown in Figure 1 of the present invention, and as disclosed and demonstrated in EP2470230.

As previously stated, the success of glaucoma surgery (whether perforating or non-perforating) is due to the fact that it limits fibrosis of the scleral and conjunctival area, and maintains the patency (and consequently the filtration capacity) of the filtration routes and the filtration/decompression bleb created in the sclera and/or in the subconjunctival space.

The use of gels based on crosslinked HA currently contributes to surgical success, but the application of HA crosslinked with BDDE (HEALA*flow*®) has not proved useful in significantly limiting/reducing the administration of MMC during glaucoma surgery (Roy S. et al., Eur J Ophthalmol, 2012, 22(1):70).

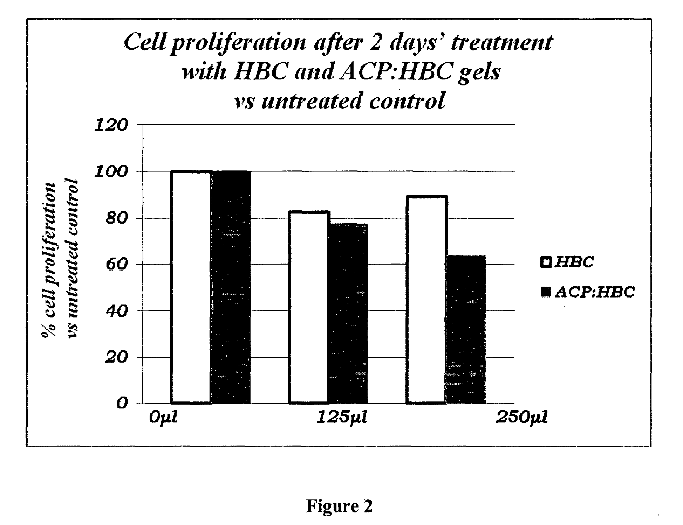

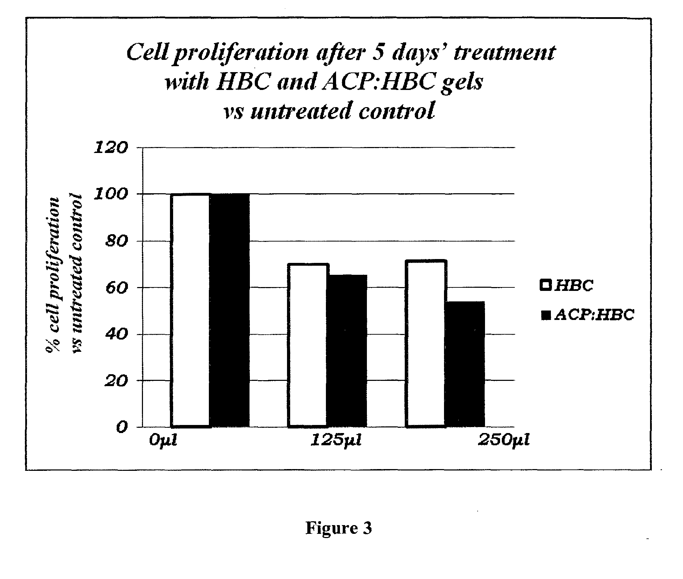

The Applicant has perfected the use of the single-phase viscoelastic gel/hydrogel ACP:HBC for use in ophthalmic surgery, in particular in glaucoma surgery, because (as demonstrated in Figures 2 and 3 below) it has discovered that said gel has a significant effect (compared with known gels based on HA and BDDE used as controls) in reducing fibrosis after both perforating and non-perforating glaucoma surgery, and is wholly devoid of cytotoxicity (as indicated by Figure 4), thus enabling treatment with antimetabolites to be eliminated (or at any rate limited).

This result is particularly important in view of the known post-operative complications associated with the use of antimetabolites (as described above), because the use of the gels produces the same anti-fibrosis efficacy as said medicaments, without their toxic effects.

Finally, Example 14 below shows that the single-phase viscoelastic gels of the invention ensure the correct filtration function of the channels treated, as they have rheological characteristics wholly comparable with those of the products based on HA crosslinked with BDDE currently on the market.

The present invention therefore claims single-phase viscoelastic gels/hydrogels formed by autocrosslinked hyaluronic acid (ACP) mixed with hyaluronic acid crosslinked with BDDE (HBC) in an ACP:HBC weight ratio ranging from 5:95 to 50:50, preferably from 5:95 to 25:75, most preferably an ACP:HBC weight ratio of 25:75, for use as anti-fibrosis gels for applications in ophthalmic surgery, in particular in all types of glaucoma surgery (both perforating and non-perforating), in the technique known as "needling of the bleb" or for the positioning of drainage implants.

The present invention therefore relates to a pharmaceutical composition containing single-phase viscoelastic gels/hydrogels formed by autocrosslinked hyaluronic acid (ACP) mixed with hyaluronic acid crosslinked with BDDE (HBC) in an ACP:HBC weight ratio ranging from 5:95 to 50:50, preferably from 5:95 to 25:75, most preferably an ACP:HBC weight ratio of 25:75, containing pharmaceutically acceptable carriers and/or excipients, for use as anti-fibrosis compositions for applications in ophthalmic surgery, in particular for applications in glaucoma surgery, optionally combined with anti-inflammatories, antibiotics, antimetabolites, local anaesthetics, or drugs able to normalise the ocular pressure.

Some preparation examples of the viscoelastic gels of the invention are set out below:

EXAMPLE 1: Synthesis of HBC (4.5%) 25 mg/ml

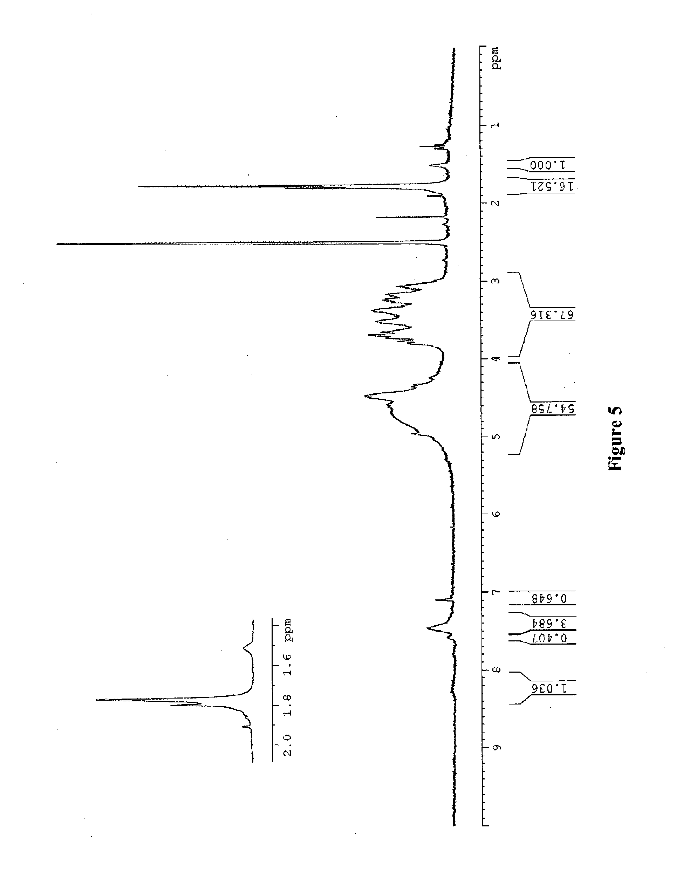

0.01 moles of HA sodium salt with a weight-average MW ranging from 500 to 750 KDa were dispersed in 30 ml of 0.25M NaOH containing 0.196 ml of BDDE (10% in moles compared with the dimer GlcUA - GlcNAc). The mixture was heated to 42°C and left to react for 4.5 hours. The resulting gel was then hydrated at room temperature for 24 hours with 75 ml of 0.1M HCl and 55 ml of water containing 1.0 g of NaCl, and the pH was adjusted to 5-6 with 0.1M HCl. After stirring, a homogenous gel was produced (final volume: 160 ml), and analysed by NMR. Figure 5 shows the $^1$H NMR spectrum

(Bruker Advance 300 MHz) in DMSO-d6, processed with XWinNMR software. Result: 4.5% moles/mole of BDDE per dimer GlcUA - GlcNAc (1/16,521x3H/4H x 100).

EXAMPLE 2: Synthesis of HBC (2.4%) 25 mg/ml

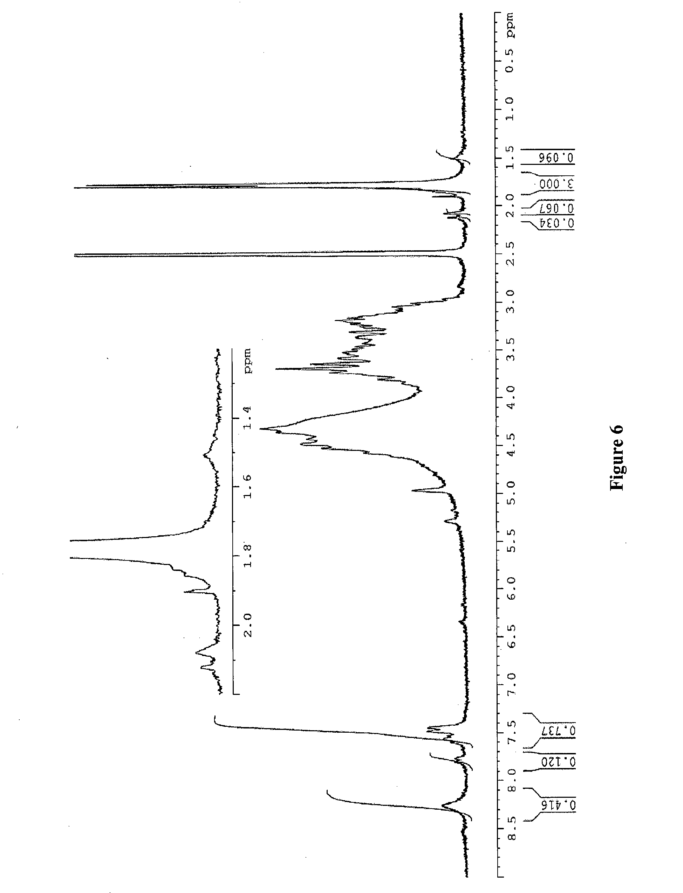

0.01 moles of HA sodium salt with a weight-average MW ranging from 500 to 750 KDa were dispersed in 30 ml of 0.25M NaOH containing 0.098 ml of BDDE (5% in moles compared with the dimer GlcUA - GlcNAc). The mixture was heated to 42°C and left to react for 4.5 hours. The resulting gel was then hydrated at room temperature for 24 hours with 75 ml of 0.1M HCl and 55 ml of water containing 1.0 g of NaCl, and the pH was adjusted to 5-6 with 0.1M HCl. After stirring, a homogenous gel was produced (final volume: 160 ml), and analysed by NMR. Figure 6 shows the $^1$H NMR spectrum (Bruker Advance 300 MHz) in DMSO-d6, processed with XWinNMR software. Result: 2.4% moles/mole of bonded BDDE per dimer GlcUA - GlcNAc (0.096/4H x 100).

EXAMPLE 3: Synthesis of HBC (6.18%) 25 mg/ml

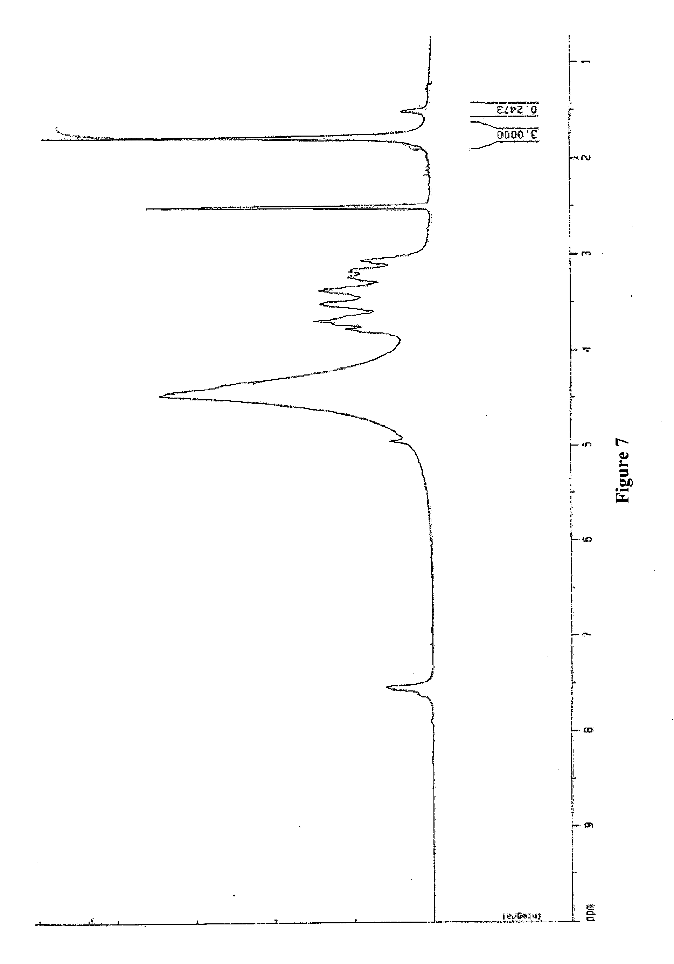

0.01 moles of HA sodium salt with a weight-average MW ranging from 500 to 750 KDa were dispersed in 30 ml of 0.25M NaOH containing 0.294 ml of BDDE (15% in moles compared with the dimer GlcUA - GlcNAc). The mixture was heated to 42°C and left to react for 5 hours. The resulting gel was then hydrated at room temperature for 24 hours with 75 ml of 0.1M HCl and 55 ml of water containing 1.0 g of NaCl, and the pH was adjusted to 5-6 with 0.1M HCl. After stirring, a homogenous gel was produced (final volume: 160 ml), and analysed by NMR. Figure 7 shows the $^1$H NMR spectrum (Bruker Advance 300 MHz) in DMSO-d6. Processed with XWinNMR software. Result: 6.18% moles/mole of BDDE per dimer GlcUA - GlcNAc (0.2473/4H x 100).

EXAMPLE 4: Preparation of ACP gel

6.5 grams (10 millimoles) of hyaluronic acid tetrabutylammonium salt, prepared from HA sodium salt as described in EP216453, were solubilised in 260 ml of N-methyl-2-pyrrolidone (NMP) at room temperature. 1.4 ml (10 millimoles) of triethylamine were then added, and the resulting solution was stirred for 30 minutes. 0.256 grams of 2-chloro-1-methyl-pyridinium-iodide (CMPJ), equal to 10% of the initial moles of HA, dissolved in 2.5 ml of NMP, were added. The solution was maintained under stirring for 4 hours at room temperature, then mixed with a 2.5% saline solution of NaCl in water (w/w). The resulting mixture was slowly poured into 750 ml of 95% ethanol and maintained under continuous stirring. The precipitate formed was then filtered and washed three times with 100 ml of ethanol/water (9:1 ratio), and twice more with 100 ml of acetone, and finally dried under high vacuum for 24 hours at 30°C. 3.9 gm of the desired product, with 4.5-5% crosslinking, was thus obtained.

EXAMPLE 5: Preparation of ACP:HBC gel in the 25:75 ratio, with a total HA concentration of 25 mg/ml

0.0033 moles of ACP powder, prepared as in Example 4, were swollen overnight in 53 ml of saline solution. They were then mixed with 160 ml of HBC (4.5%) 25 mg/ml prepared as described in Example 1, for 3 hours at room temperature, and the homogenous gel thus obtained was filtered through 100 μm cartridges. Finally, the sample thus prepared was distributed in 1 ml glass syringes and sterilised in the autoclave with a cycle of F0=13 at 121.5°C. The resulting gel is a single-phase gel with a zero shear viscosity of 2418 Pas.

The analysis was conducted on a Thermo Haake Mars II rheometer at 25°C using a cone/plate (6 cm diameter plate and 1° angle), rotating from 1000 to 0.0005 s[-1]. The residual BDDE was less than 2 ppm (analysis conducted by GC vs a standard).

EXAMPLE 6: Preparation of ACP:HBC gel in the 50:50 ratio,with a total HA concentration of 25 mg/ml

0.0033 moles of ACP powder, prepared as in Example 4, were swollen overnight in 53 ml of saline solution. The ACP was then mixed with 53 ml of HBC (4.5%) 25 mg/ml, prepared as described in Example 1, for 3 hours at room temperature, and the homogenous gel thus obtained was filtered through 100 μm cartridges. Finally, the sample, distributed in 1 ml glass syringes, was sterilised in the autoclave with a cycle of F0=13 at 121.5°C. The product obtained is a single-phase gel with a BDDE content of less than 2 ppm (analysis conducted by GC vs a standard).

EXAMPLE 7: Preparation of ACP:HBC gel in the 5:95 ratio,  with a total HA concentration of 25 mg/ml

0.00052 moles of ACP powder, prepared as in Example 4, were swollen overnight in 8.4 ml of saline solution. The ACP was then mixed with 160 ml of HBC (4.5%) 25 mg/ml, prepared as described in Example 1, for 3 hours at room temperature, and the homogenous gel thus obtained was filtered through 100 μm cartridges. Finally, the sample, distributed in 1 ml glass syringes, was sterilised in the autoclave with a cycle of F0=13 at 121.5°C. The preparation obtained is a single-phase gel with a zero shear viscosity of 3566 Sbp (the analysis was conducted as described in Example 5), and a BDDE content of less than 2 ppm (analysis conducted by GC vs a standard).

EXAMPLE 8: Preparation of ACP:HBC gel in the 5:95 ratio, with a total HA concentration of 25 mg/ml

0.00052 moles of ACP powder, prepared as in Example 4, were swollen overnight in 8.4 ml of saline solution. The ACP was then mixed with 160 ml of HBC (6.18%) 25 mg/ml, prepared as described in Example 3, for 3 hours at room temperature, and the homogenous gel thus obtained was filtered through 100 μm cartridges. Finally, the sample, distributed in 1 ml glass syringes, was sterilised in the autoclave with a cycle of F0=13 at 121.5°C. The preparation obtained is a single-phase gel with a BDDE content of less than 2 ppm (analysis conducted by GC vs a standard).

EXAMPLE 9: Preparation of ACP:HBC gel in the 5:95 ratio, with a total HA concentration of 15 mg/ml

96 ml of HBC (4.5%) 25 mg/ml, prepared as described in Example 1, were mixed with 64 ml of saline overnight at room temperature, until 160 ml of a gel at the HA concentration of 15 mg/ml was obtained. 0.00031 moles of ACP powder, prepared as in Example 4, were swollen overnight in 8.4 ml of saline solution. The ACP was then mixed for 3 hours at room temperature with 160 ml of said HBC, and the homogenous gel thus obtained was filtered through 100 μm cartridges. Finally, the sample, distributed in 1 ml glass syringes, was sterilised in the autoclave with a cycle of F0=13 at 121.5°C. The product obtained is a single-phase gel with a BDDE content of less than 2 ppm (analysis conducted by GC vs a standard).

EXAMPLE 10: Preparation of ACP:HBC gel (6.18%) in the 5:95 ratio, total HA concentration 25 mg/ml, with 3 mg/ml lidocaine, in phosphate buffer pH 7.0

0.005 moles of HA sodium with a weight-average MW ranging from 500 to 750 KDa were dispersed in 15 ml of 0.25M NaOH containing 0.147 ml of BDDE (15% in moles compared with the dimer GlcUA - GlcNAc). The mixture was heated to 42°C and left to react for 4.5 hours. The gel was then hydrated at room temperature for 24 hours with 37 ml of 0.1M HCl and 28 ml of $H_2O$ containing 0.27 gm of lidocaine chloride, 0.47 gm of NaCl, 0.042 gm of $Na_2HPO_4 12H_2O$ and 0.0076 mg of $NaH_2PO_4 2H_2O$, adjusting the pH to 5-6 with 0.1M HCl. After stirring, the homogenous gel (with a final volume of 80 ml) thus obtained was mixed with 4.2 ml of ACP gel (obtained by swelling 0.00025 moles of ACP powder, prepared as described in Example 4, in 4.2 ml of $H_2O$ for 10 hours), and left at 70°C for 7 hours to eliminate the BDDE content. The product thus prepared was then mixed, filtered through 100 μm cartridges and finally distributed in 1 ml glass syringes and sterilised in the autoclave with a cycle of F0=13 at 121.5°C. The final sample is a single-phase viscoelastic gel with a BDDE content of less than 2 ppm (analysis conducted by GC vs a standard).

EXAMPLE 11

Eye irritation test according to guidelines (ISO 10993-10) on 2 HA products crosslinked with BDDE mixed with ACP, such as:

1. ACP:HBC in the 25:75 ratio prepared in saline, as described in Example 5;
2. ACP:HBC in the 5:95 ratio prepared in phosphate buffer pH 7.0, as described in Example 10.

The eye irritation test was conducted at the PRIMM srl Test Centre Laboratories (Dosson di Casier (TV), Italy), by ocular administration of said 2 products, each product being evaluated on 3 animals.

The test was performed on the NZW laboratory rabbit (the NZW rabbit is considered to be the model animal most suitable for intraconjunctival administration of products with ocular action).

The two test products were administered intraconjunctivally; the eye of the treated animals was then examined 1 hour after administration and 24, 48 and 72 hours thereafter, and compared with the untreated contralateral eye which acted as reference control.

The observations conducted were:

a) clinical state of conjunctiva,
b) clinical state of iris and cornea.

Results: for each animal and each observation period, the evaluations conducted gave a macroscopic indication of excellent clinical tolerability, and did not indicate any difference from the untreated control.

EXAMPLE 12: Cutaneous filling and residence time of ACP:HBC gel in the intradermal administration model in the rabbit

The purpose of the experiment was to evaluate the cutaneous filling and total residence time *in situ* determined by the ACP:HBC gel prepared as described in Example 5, injected into the intradermal tissue of the rabbit, by comparison with the reference gel consisting of HA crosslinked with equal amounts of BDDE (control gel).

For said evaluation, the gels tested were administered intradermally to male NZW-KBL rabbits weighing 1.8-2.3 kg.

**Experiment design:**

The animals were anaesthetised by intravenous administration of ketamine and xylazine. 3 animals were used for each sample tested.

Day 0: T0

- Injection of samples (1 ml of gel per sample) after shaving the rabbits' backs;
- Measurement of the swelling on all rabbits and macroscopic observation for adverse events.

Day 28 and 85: T28 and T85

- Measurement of swelling volume and macroscopic observation for adverse events.

The swelling volume was calculated with the formula:

$$(2/3 \times \pi) \times (r1) \times (r2) \times (r3)$$

wherein: (r1), (r2) and (r3) represent the width, length and height of the swelling respectively, measured with a caliper.

Results:

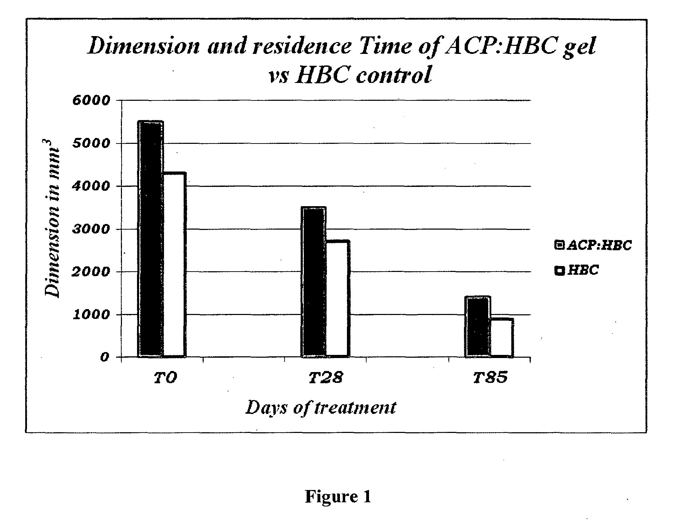

The viscoelastic gel ACP:HBC did not cause any inflammatory event in the treated dermis; the results for residence time *in situ* are shown in Figure 1; the size of the initial swelling (expressed as mm$^3$) demonstrated that the gel according to the invention is capable of inducing a larger skin swelling volume than the control gel, which remains significantly high even after 28 and 85 days. As previously demonstrated in EP2470230, this effect is attributable to the presence of the ACP derivative which, in view of its chemical/rheological characteristics, has proved essential to promote immediate and stable cutaneous filling.

EXAMPLE 13: evaluation of anti-fibrosis efficacy of ACP:HBC gel

The purpose of the test was to evaluate the antifibrotic efficacy of the gels according to the invention, at the same time testing their cytotoxicity.
The ACP:HBC gel produced as described in Example 5 was used for this purpose, by comparison with the reference product represented by the gel formed by HA crosslinked with equal amounts of BDDE, both tested at the same total HA concentration.

Operational procedure

To test the activity of the gels described above *in vitro,* the cell viability/proliferation tests were performed with human connective tissue cells seeded in wells (100,000 cells/well) incubated with the ACP:HCB and HCB samples at the concentrations of 125 $\mu$l and 250 $\mu$l of gel/ml of medium, for incubation times of 2 and 5 days, in an incubator at 37°C.
Preparation of cell cultures: briefly, human connective tissue obtained from biopsies, after several washes in PBS (Phosphate Buffered Solution) with the addition of antibiotics, is cut into small strips which are digested with trypsin for 10-20 minutes at 37°C. At the end of the treatment the cells are extracted by centrifugation and seeded in a culture medium consisting of DMEM supplemented with 20% foetal bovine serum, 1% P/S (penicillin/streptomycin) and 1% glutamine.

After 2 and 5 days' treatment, the MTT test was conducted to evaluate the effect of the samples on cell proliferation/viability.

MTT TEST

The MTT test quantitatively measures the presence of succinate dehydrogenase activity in the cell cultures. The activity of this enzyme, which is only present in the mitochondria of viable cells, is normally used as a marker to investigate the metabolic activity, viability and/or growth of cell cultures. The test is based on the conversion of the chemical compound MTT (3-(4,5-dimethylthiazol-2-yl)-2,5 diphenyltetrazolium bromide), a yellow azo dye, to blue formazan salts, by mitochondrial succinate dehydrogenase. The quantity of formazan determined spectrophotometrically is proportional to the presence of said enzyme in the cell culture, and is therefore directly proportional to the number of viable cells. The cells are incubated with 0.5 mg/ml MTT solution for 3 hours. At the end of incubation the dye is extracted from the viable cells with an extraction solution consisting of 90% isopropanol and 10% DMSO. The dye is then read at a wavelength, O.D., of 540 nm.

Results

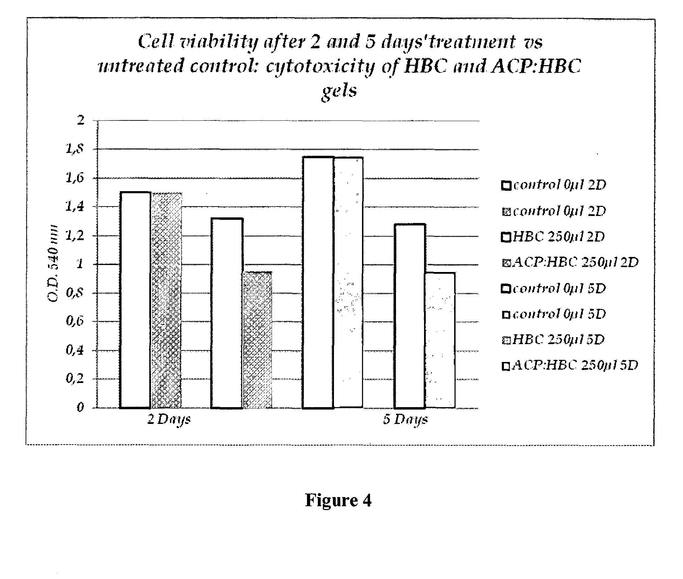

The results clearly demonstrate that after 2 days' treatment, the ACP:HBC gel inhibited cell proliferation by nearly 40% vs the untreated control (at the highest concentration tested), whereas the HBC gel determined a response of just over 10% inhibition. After 5 days' treatment the % inhibition increased to 50% of the control, whereas the reference gel remained at around 30%.
Finally, Figure 4 shows that said gels are wholly devoid of cytotoxicity, even at the highest dose tested; even after 5 days' treatment, the viability of the cells remains unchanged, while their proliferative capacity is inhibited.
These findings confirm what has been previously stated: the viscoelastic gels according to the invention significantly reduce (vs comparative gels containing HA crosslinked with BDDE) cell proliferation in the scleral area (i.e. connective tissue cells) subjected to ophthalmic surgery, but without being cytotoxic, thus allowing the surgeon to use said gels without simultaneously treating the area concerned with antiproliferative drugs, or at any rate allowing a reduction in the quantity used.

EXAMPLE 14: rheological properties

The viscoelastic gels of the invention ensure the correct filtration function of the channels treated and the filtration bleb created, because they possess rheological characteristics wholly comparable with those of the products based on HA crosslinked with BDDE currently on the market for glaucoma surgery.
HA exhibits both viscous and elastic properties that mainly depend on its molecular weight. Said viscoelastic characteristics can be quantified by measuring its moduli; the first modulus is called G' or modulus of elasticity, because it represents the energy stored when the polysaccharide is subjected to a tension/deformation, and the second modulus is called G" or modulus of viscosity, because it represents the energy dissipated when the molecule is subjected to a tension.

The measurements of G' and G" were taken at 25°C with a Thermo Haake Mars II (cone/plate) rheometer equipped with a 6 cm diameter cone and an angle of 1°. The measurements were taken in oscillating mode, in a frequency range measured from 0.001 to 1000 rad/s. The samples were processed with Haake Rheowin Job Manager 4.0 software.

Results

ACP:HBC, prepared and sterilised as described in Example 5, at the final concentration of 25 mg/ml in HA:

$$G' \text{ at } 0{,}628 \text{ rad/s} = 31.4 \text{ Pa}$$

$$G'' \text{ at } 0{,}628 \text{ rad/s} = 12.9 \text{ Pa}$$

HBC, prepared and sterilised as for HBC in the previous sample, at the same concentration of 25 mg/ml in HA:

$$G' \text{ at } 0{,}628 \text{ rad/s} = 32.82 \text{ Pa}$$

$$G'' \text{ at } 0{,}628 \text{ rad/s} = 11.95 \text{ Pa}$$

The results obtained prove that the two moduli of ACP:HBC gel are comparable with those of the control gel (representative of the product based on HA crosslinked with BDDE currently on the market for glaucoma), thus demonstrating that the gels have the same viscosity and elasticity, and therefore the same filtration capacity.

Basically, these findings confirm that the combination of the ACP gel and the HBC gel increases the residence time *in situ* of said gel but especially reduces the fibrosis in the treated area and maintains unchanged the filtration efficiency of the gel of the invention.

**Claims**

1. Viscoelastic gels and hydrogels made of autocrosslinked hyaluronic acid (ACP) mixed with hyaluronic acid crosslinked with 1,4-butanediol diglycidyl ether, BDDE (HBC) in a weight ratio from 5:95 to 50:50, for use in ophthalmic surgery as antifibrotic gels.

2. Viscoelastic gels and hydrogels for use according to claim 1 for applications in every kind of glaucoma surgery, both penetrating and non-penetrating, and in "needling of the bleb" or for positioning drainage implants.

3. Viscoelastic gels and hydrogels for use according to any one of claims 1-2, preferably in an ACP:HBC weight ratio from 5:95 to 25:75, most preferably of 25:75.

4. Viscoelastic gels and hydrogels for use according to any one of claims 1-3, wherein the hyaluronic acid used for the preparation of ACP and HBC derivatives is preferably a hyaluronic acid sodium salt and has a weight-average molecular weight from 400 to $3 \times 10^6$ Da, preferably from $1 \times 10^5$ Da to $1 \times 10^6$ Da, most preferably from 200,000 to 750,000 Da.

5. Viscoelastic gels and hydrogels for use according to any one of claims 1-4 in the ACP:HBC weight ratio of 25:75, wherein the hyaluronic acid has a weight-average molecular weight from 200,000 to 750,000 Da.

6. Viscoelastic gels and hydrogels for use according to any one of claims 1-5, wherein ACP has a mean degree of crosslinking from 4% to 5%, and is prepared with hyaluronic acid having a weight-average molecular weight of 200 KDa.

7. Viscoelastic gels and hydrogels for use according to any one of claims 1-6, wherein HBC has a BDDE/hyaluronic acid molar ratio from 2% to 7% moles/mole of hyaluronic acid dimer, preferably from 4% to 5% moles/mole of dimer.

8. Viscoelastic gels and hydrogels for use according to any one of claims 1-7, wherein hyaluronic acid has a total concentration from 10 to 40 mg/ml, preferably from 20 to 30 mg/ml.

9. Viscoelastic gels and hydrogels for use according to any one of claims 5-8, in the ACP:HBC weight ratio of 25:75, wherein ACP possesses a mean degree of crosslinking from 4% to 5% and is prepared starting from a hyaluronic acid having a weight-average molecular weight of 200 KDa, HBC has a BDDE/hyaluronic acid molar ratio from 4% to 5% moles/mole of hyaluronic acid dimer, and the total concentration of hyaluronic acid ranges from 20 to 30 mg/ml, for use in applications in every kind of glaucoma surgery as antifibrotic gels.

10. Pharmaceutical compositions comprising viscoelastic gels and hydrogels made of autocrosslinked hyaluronic acid (ACP) mixed with hyaluronic acid crosslinked with BDDE (HBC) in a weight ratio from 5:95 to 50:50, preferably from 5:95 to 25:75 most preferably of 25:75, containing pharmaceutically acceptable carriers and/or excipients, for use in ophthalmic surgery as antifibrotic compositions.

11. Pharmaceutical compositions for use according to claim 10, for use specifically in glaucoma surgery as antifibrotic compositions.

12. Pharmaceutical compositions for use according to any one of claims 10-11, combined with anti-inflammatory drugs, antibiotics, antimetabolites, local anaesthetics or drugs that regulate ocular pressure.

13. Pharmaceutical compositions for use according to any one of claims 10-12 in the form of viscoelastic hydrogels wherein the pharmaceutically acceptable carriers and/or excipients are saline and physiological solutions of NaCl and/or phosphate salts.

14. Pharmaceutical compositions for use according to claim 13, having a pH ranging from 5.5 to 7.

**Patentansprüche**

1. Viskoelastische Gele und Hydrogele, hergestellt aus autovernetzter Hyaluronsäure (ACP) im Gemisch mit Hyaluronsäure, vernetzt mit 1,4-Butandioldiglycidylether BDDE, (HBC) in einem Gewichtsverhältnis von 5:95 bis 50:50, zur Verwendung als antifibrotische Gele in der Augenchirurgie.

2. Viskoelastische Gele und Hydrogele zur Verwendung gemäß Anspruch 1 für Anwendungen aller Art bei Glaukomchirurgie, sowohl penetrierende und nichtpenetrierende, und im "Sicker-kissen-Needling" oder zur Positionierung von Drainageimplantaten.

3. Viskoelastische Gele und Hydrogele zur Verwendung gemäß einem der Ansprüche 1-2, vorzugsweise in einem Gewichtsverhältnis ACP:HBC von 5:95 bis 25:75, am stärksten bevorzugt 25:75.

4. Viskoelastische Gele und Hydrogele zur Verwendung gemäß einem der Ansprüche 1-3, wobei die für die Herstellung von ACP- und HBC-Derivaten verwendete Hyaluronsäure vorzugsweise ein Hyaluronsäurenatriumsalz ist, und ein gewichtsmittleres Molekulargewicht von 400 bis $3\times10^6$ Da, vorzugsweise $1\times10^5$ Da bis $1\times10^6$ Da, am stärksten bevorzugt von 200.000 bis 750.000 Da hat.

5. Viskoelastische Gele und Hydrogele zur Verwendung gemäß einem der Ansprüche 1-4 mit einem Gewichtsverhältnis ACP:HBC von 25:75, wobei die Hyaluronsäure ein gewichtsmittleres Molekulargewicht von 200.000 bis 750.000 Da aufweist.

6. Viskoelastische Gele und Hydrogele zur Verwendung gemäß einem der Ansprüche 1-5, wobei das ACP einen mittleren Vernetzungsgrad von 4% bis 5% aufweist und mit Hyaluronsäure mit einem gewichtsmittleren Molekulargewicht von 200 kDa hergestellt wird.

7. Viskoelastische Gele und Hydrogele zur Verwendung gemäß einem der Ansprüche 1-6, wobei HBC ein molares Verhältnis BDDE/Hyaluronsäure von 2 Mol-% bis 7 Mol-%/Mol Hyaluronsäuredimer, vorzugsweise 4 Mol-% bis 5 Mol-%/Mol Dimer aufweist.

8. Viskoelastische Gele und Hydrogele zur Verwendung gemäß einem der Ansprüche 1-7, wobei die Hyaluronsäure eine Gesamtkonzentration von 10 bis 40 mg/ml, vorzugsweise von 20 bis 30 mg/ml besitzt.

9. Viskoelastische Gele und Hydrogele zur Verwendung gemäß einem der Ansprüche 5-8 mit einem Gewichtsverhältnis ACP:HBC von 25:75, wobei ACP einen mittleren Vernetzungsgrad von 4% bis 5% besitzt, und hergestellt wird ausgehend von einer Hyaluronsäure mit einem gewichtsmittleren Molekulargewicht von 200 kDa, HBC ein molares Verhältnis BDDE/Hyaluronsäure von 4 Mol-% bis 5 Mol-%/Mol Hyaluronsäuredimer aufweist, und die Gesamtkonzentration von Hyaluronsäure von 20 bis 30 mg/ml reicht, zur Verwendung in Anwendungen aller Art bei Glaukomoperation als antifibrotische Gele.

10. Pharmazeutische Zusammensetzungen, umfassend viskoelastische Gele und Hydrogele, hergestellt aus autovernetzter Hyaluronsäure (ACP) im Gemisch mit Hyaluronsäure, vernetzt mit BDDE, (HBC) in einem Gewichtsverhältnis von 5:95 bis 50:50, bevorzugt 5:95 bis 25:75, am stärksten bevorzugt 25:75, enthaltend pharmazeutisch annehmbare Träger und/oder Exzipienzien, zur Verwendung als antifibrotische Zusammensetzungen der Augenchirurgie.

11. Pharmazeutische Zusammensetzungen zur Verwendung gemäß Anspruch 10, zur besonderen Verwendung als antifibrotische Zusammensetzungen in der Glaukomchirurgie.

12. Pharmazeutische Zusammensetzungen zur Verwendung gemäß einem der Ansprüche 10-11 in Kombination mit entzündungshemmenden Wirkstoffen, Antibiotika, Antimetaboliten, Lokalanästhetika oder Wirkstoffen, die den Augendruck regulieren.

**13.** Pharmazeutische Zusammensetzungen zur Verwendung gemäß einem der Ansprüche 10-12 in Form viskoelastischer Hydrogele, wobei die pharmazeutisch annehmbaren Träger und/oder Exzipienzien physiologische Kochsalzlösungen und physiologische Lösungen von NaCl und/oder Phosphatsalzen sind.

**14.** Pharmazeutische Zusammensetzungen zur Verwendung gemäß Anspruch 13 mit einem pH, der von 5,5 bis 7 reicht.


**Revendications**

**1.** Gels et hydrogels viscoélastiques réalisés à partir d'acide hyaluronique autoréticulé (ACP) mélangé avec de l'acide hyaluronique réticulé avec de l'éther diglycidylique de 1,4-butanediol BDDE (HBC) dans un rapport pondéral de 5:95 à 50:50, pour leur utilisation dans la chirurgie ophtalmique à titre d'agents antifibrotiques.

**2.** Gels et hydrogels viscoélastiques pour leur utilisation selon la revendication 1, pour des applications dans tout type de chirurgie du glaucome, à la fois de type pénétrant et non pénétrant, et dans la « ponction de la bulle à l'aiguille », ou pour le positionnement d'implants de drainage.

**3.** Gels et hydrogels viscoélastiques pour leur utilisation selon l'une quelconque des revendications 1-2, de préférence dans un rapport pondéral ACP:HBC de 5:95 à 25:75, de manière de loin préférée de 25:75.

**4.** Gels et hydrogels viscoélastiques pour leur utilisation selon l'une quelconque des revendications 1-3, dans lesquels l'acide hyaluronique utilisé pour la préparation des dérivés ACP et HBC représente de préférence un sel de sodium de l'acide hyaluronique et possède un poids moléculaire moyen en poids de 400 à $3 \times 10^6$ Da, de préférence de $1 \times 10^5$ Da à $1 \times 10^6$ Da, de manière de loin préférée de 200.000 à 750.000 Da.

**5.** Gels et hydrogels viscoélastiques pour leur utilisation selon l'une quelconque des revendications 1-4, dans le rapport pondéral ACP:HBC de 25:75, dans lesquels l'acide hyaluronique possède un poids moléculaire moyen en poids de 200.000 à 750.000 Da.

**6.** Gels et hydrogels viscoélastiques pour leur utilisation selon l'une quelconque des revendications 1-5, dans lesquels l'ACP possède un degré moyen de réticulation de 4 % à 5 %, et est préparé avec de l'acide hyaluronique possédant un poids moléculaire moyen en poids de 200 KDa.

**7.** Gels et hydrogels viscoélastiques pour leur utilisation selon l'une quelconque des revendications 1-6, dans lesquels le HBC possède un rapport molaire BDDE/acide hyaluronique de 2 mol % à 7 mol %/mole d'acide hyaluronique dimère, de préférence de 4 mol % à 5 mol %/mole de dimère.

**8.** Gels et hydrogels viscoélastiques pour leur utilisation selon l'une quelconque des revendications 1-7, dans lesquels l'acide hyaluronique possède une concentration totale de 10 à 40 mg/ml, de préférence de 20 à 30 mg/ml.

**9.** Gels et hydrogels viscoélastiques pour leur utilisation selon l'une quelconque des revendications 5-8, dans le rapport pondéral ACP:HBC de 25:75, dans lesquels l'ACP possède un degré moyen de réticulation de 4 % à 5 %, et est préparé à partir d'un acide hyaluronique possédant un poids moléculaire moyen en poids de 200 KDa, le HBC possède un rapport molaire BDDE/acide hyaluronique de 4 mol % à 5 mol %/mole d'acide hyaluronique dimère, et la concentration totale de l'acide hyaluronique se situe dans la plage de 20 à 30 mg/ml, pour leur utilisation dans des applications dans tout type de chirurgie du glaucome à titre de gels antifibrotiques.

**10.** Compositions pharmaceutiques comprenant des gels et hydrogels viscoélastiques réalisés à partir d'acide hyaluronique autoréticulé (ACP) mélangé avec de l'acide hyaluronique réticulé avec du BDDE (HBC) dans un rapport pondéral de 5:95 à 50:50, de préférence de 5:95 à 25:75, de manière de loin préférée de 25:75, contenant des supports et/ou des excipients pharmaceutiquement acceptables, pour leur utilisation dans la chirurgie ophtalmique à titre de compositions antifibrotiques.

**11.** Compositions pharmaceutiques pour leur utilisation selon la revendication 10, pour leur utilisation de manière spécifique dans la chirurgie du glaucome à titre de compositions antifibrotiques.

**12.** Compositions pharmaceutiques pour leur utilisation selon l'une quelconque des revendications 10-11, en combinaison avec des médicaments anti-inflammatoires, des antibiotiques, des antimétabolites, des anesthésiques locaux ou des médicaments qui régulent la pression oculaire.

**13.** Compositions pharmaceutiques pour leur utilisation selon l'une quelconque des revendications 10-12, sous la forme d'hydrogels viscoélastiques, dans lesquelles les supports et/ou les excipients pharmaceutiquement acceptables sont des solutions salines et physiologiques de NaCl et/ou de sels phosphate.

**14.** Compositions pharmaceutiques pour leur utilisation selon la revendication 13, possédant un pH qui se situe dans la plage de 5,5 à 7.

Figure 1

Figure 2

Figure 3

Figure 4

Figure 5

Figure 6

**Figure 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100069938 A
- EP 2470230 A
- EP 0341745 A
- EP 0138572 A
- WO 2012032154 A
- EP 216453 A

**Non-patent literature cited in the description**

- **FOSCARINI B. et al.** *ARVO 2012 Annual Meeting Abstracts,* 2012
- **TERBOJEVICH et al.** *Carbohydrate Research,* 1986, vol. 149, 363-377
- **ROY S. et al.** *Eur J Ophthalmol,* 2012, vol. 22 (1), 70